(19) Europäisches Patentamt
European Patent Office
Office européen des brevets

(11) **EP 4 553 710 A1**

(12) **EUROPEAN PATENT APPLICATION**
published in accordance with Art. 153(4) EPC

(43) Date of publication:
**14.05.2025 Bulletin 2025/20**

(21) Application number: **23857715.9**

(22) Date of filing: **23.08.2023**

(51) International Patent Classification (IPC):
**G06N 3/08** (2023.01)    **G06N 3/042** (2023.01)
**G06N 3/063** (2023.01)

(52) Cooperative Patent Classification (CPC):
**G06N 3/042; G06N 3/063; G06N 3/08**

(86) International application number:
**PCT/KR2023/012469**

(87) International publication number:
**WO 2024/043683 (29.02.2024 Gazette 2024/09)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.08.2022 KR 20220105457**

(71) Applicant: **LG Management Development Institute
Co., Ltd.
Seoul 07336 (KR)**

(72) Inventors:
• **RODRIGO, Hormazabal
Seoul 07796 (KR)**
• **HWANG, Doyeong
Seoul 07524 (KR)**
• **KIM, Kiyoung
Seoul 07811 (KR)**
• **HAN, Se Hui
Seoul 07665 (KR)**
• **LEE, Honglak
Seoul 07796 (KR)**

(74) Representative: **BCKIP Part mbB
MK1
Landsbergerstraße 98, 3.Stock
80339 München (DE)**

(54) **DEVICE FOR IMPLEMENTING SEQUENCE TRANSDUCTION NEURAL NETWORK FOR TRANSDUCING INPUT SEQUENCE, AND TRAINING METHOD USING SAME**

(57)    A neural network implementation device according to an embodiment of the present invention may comprise: at least one memory; and at least one processor that communicates with the at least one memory, wherein the at least one processor is implemented to: receive first input data; receive second input data corresponding to the first input data; train a sequence transduction neural network by performing an attention operation on the basis of the first input data and second input data labeled with predetermined label information; and determine output data that is output by the sequence transduction neural network trained on the basis of the first input data, the second input data, and the label information.

[Fig. 2]

## Description

[Technical Field]

The present disclosure relates to a device and method for predicting a neoantigen. More specifically, the present disclosure relates to a device for implementing a sequence transduction neural network for transducing an input sequence, and a training method using the same.

[Background Art]

Recently, various concepts and models (neural networks) have been developed in the field of artificial intelligence technology, and research on data prediction using the same has been actively conducted.

However, when predicting data based on an artificial intelligence-based neural network, it is necessary to develop training or prediction algorithms for the models to derive more accurate results, that is, results with a higher prediction probability.

[Disclosure]

[Technical Problem]

Embodiments disclosed in the present disclosure are directed to providing an algorithm that may derive more accurate results when predicting data based on an artificial intelligence-based neural network, and further, providing a device and method for extracting a neoantigen candidate using an artificial intelligence-based neoantigen prediction model that may provide immune anticancer vaccine treatment to a patient by facilitating prediction of a neoantigen using the algorithm.

Objects of the present disclosure are not limited to the above-described object, and other objects that are not mentioned will be clearly understood by those skilled in the art from the following description.

[Technical Solution]

A neural network implementation device for implementing a sequence transduction neural network for transducing an input sequence having each network input for at least one computer according to the present disclosure to achieve the above-described object may include at least one memory, and at least one processor configured to communicate with the at least one memory, wherein the at least one processor may be configured to receive first input data, receive second input data corresponding to the first input data, train a sequence transduction neural network by performing an attention operation on the basis of the first input data and second input data labeled with predetermined label information, and determine output data that is output by the sequence transduction neural network trained on the basis of the first input data, the second input data, and the label information.

In addition, a training method for transducing an input sequence performed by a sequence transduction neural network on a computer device according to the present disclosure may include performing a predetermined pre-training operation on the basis of first input data to determine a first key value and a first value value, matching position information to each sequence of second input data when the second input data corresponding to the first input data is input, performing a predetermined self attention operation on the basis of a second key value, a second value value, and a second query value that correspond to the second input data to generate a first query value, and performing the predetermined attention operation on the basis of the first key value, the first value value, and the first query value to determine the output data corresponding to the first input data and the second input data.

In addition, a computer program stored in a computer-readable recording medium for implementing the present disclosure may further be provided.

In addition, a computer-readable recording medium configured to record a computer program for implementing the present disclosure may further be provided.

[Advantageous Effects]

According to the above-described technical solution of the present disclosure, an algorithm that can derive more accurate results when predicting data based on an artificial intelligence-based neural network can be provided, and further, immune anticancer vaccine treatment can be provided to a patient by facilitating prediction of a neoantigen using the algorithm.

Effects of the present disclosure are not limited to the above effect, and other effects that are not mentioned will be clearly understood by those skilled in the art from the following description.

[Description of Drawings]

FIG. 1 is a diagram schematically showing a training operation for implementing a sequence transduction neural network according to one embodiment of the present disclosure.

FIG. 2 is a diagram schematically showing an attention algorithm of the sequence transduction neural network implemented according to one embodiment of the present disclosure.

FIG. 3 is a diagram showing an example of performing a scaled dot attention operation in the attention algorithm of the sequence transduction neural network implemented according to one embodiment of the present disclosure.

FIG. 4 is a diagram showing an example of processing second input data in the attention algorithm of the sequence transduction neural network implemented according to one embodiment of the present disclosure.

FIG. 5 is a diagram showing an example of processing first input data in the attention algorithm of the sequence transduction neural network implemented according to one embodiment of the present disclosure.

FIG. 6 is a diagram showing an example of generating output data using an attention score value calculated in the attention algorithm of the sequence transduction neural network implemented according to one embodiment of the present disclosure.

FIG. 7 is a diagram showing a configuration of a computer device for transducing an input sequence according to one embodiment of the present disclosure.

FIG. 8 is a diagram showing a specific configuration of a neural network implemented in a processor of the computer device of FIG. 7.

FIG. 9 is a flowchart showing an example of processing first input data based on the attention algorithm of the sequence transduction neural network implemented according to one embodiment of the present disclosure.

FIG. 10 is a flowchart showing an example of processing second input data based on the attention algorithm of the sequence transduction neural network implemented according to one embodiment of the present disclosure.

FIG. 11 is a flowchart showing an example of generating output data using an attention score value calculated based on the attention algorithm of the sequence transduction neural network implemented according to one embodiment of the present disclosure.

FIGS. 12 and 13 are flowcharts showing examples of predicting whether to bind a peptide sequence to major histocompatibility complexes (MHC) and the activation of a T-cell based on the attention algorithm of the sequence transduction neural network implemented according to one embodiment of the present disclosure.

[Modes of the Invention]

Advantages and features of the present disclosure and methods of achieving them will become clear by referring to the embodiments described below in detail in conjunction with the accompanying drawings. However, the present disclosure is not limited to the embodiments disclosed below and can be implemented in various different forms, and the embodiments are merely provided to make the present disclosure complete and to fully inform those skilled in the art to which the present disclosure pertains of the scope of the present disclosure, and the present disclosure is only defined by the scope of the appended claims.

The terms used in the present specification are for describing the embodiments and are not intended to limit the present disclosure. In the present specification, the singular form also includes the plural form unless specifically stated in the phrase. The terms "comprises" and/or "comprising" that are used in the present specification do not exclude the presence or addition of one or more other components in addition to the components mentioned. The same reference numerals refer to the same components throughout the specification, and the phrase "and/or" includes each and any combination of one or more of the components mentioned. Although "first," "second," and the like are used to describe various components, it goes without saying that these components are not limited by these terms. These terms are merely used to distinguish one component from another. Therefore, it goes without saying that a first component mentioned below may also be a second component within the technical idea of the present disclosure.

Unless otherwise defined, all terms (including technical and scientific terms) used in the present specification may be used with meanings commonly understood by those skilled in the art to which the present disclosure pertains. In addition, terms defined in dictionaries that are commonly used are not ideally or excessively interpreted unless explicitly specifically defined otherwise.

The same reference numerals refer to the same components throughout the present disclosure. The present disclosure does not describe all elements of the embodiments, and common content in the art to which the present disclosure pertains or content that overlaps between the embodiments will be is omitted. Terms "unit," "module," "member," and "block" used in the specification may be implemented as software or hardware, and according to the embodiments, a plurality of "units," "modules," "members," and "blocks" may be implemented as one component, or one "unit," "module," "member," and "block" may also include a plurality of components.

Throughout the specification, when a first component is described as being "connected" to a second component, this includes not only a case in which the first component is directly connected to the second component but also a case in which the first component is indirectly connected to the second component, and the indirect connection includes connection through a wireless communication network.

In addition, when a certain portion is described as "including," a certain component, this means further including other components rather than precluding other components unless especially stated otherwise.

Throughout the specification, when a first member is described as being positioned "on" a second member, this includes both a case in which the first member is in contact with the second member and a case in which a third member is present between the two members.

Terms such as first and second are used to distinguish one component from another, and the components are not limited by the above-described terms.

A singular expression includes plural expressions unless the context clearly dictates otherwise.

In each operation, identification symbols are used for convenience of description, and the identification symbols do not describe the sequence of each operation, and each operation may be performed in a different sequence from the specified sequence unless a specific sequence is clearly described in context.

The terms used in the following description are defined as follows.

'A device for implementing a sequence transduction neural network according to the present disclosure' in the present specification includes various devices that can perform computational processing and provide results to a user. For example, the device for implementing a sequence transduction neural network according to the present disclosure may include all of a computer device, a server device, and a portable terminal, or may be one of them.

Here, the computer device may include, for example, a notebook, a desktop, a laptop, a tablet PC, a slate PC, etc., which are provided with a web browser.

The server device is a server that processes information in communication with an external device and may include an application server, a computing server, a database server, a file server, a game server, a mail server, a proxy server, and a web server.

The portable terminal is, for example, a wireless communication device ensuring portability and mobility and may include all kinds of handheld-based wireless communication devices such as a personal communication system (PCS), a global system for mobile communications (GSM), a personal digital cellular (PDC), a personal handyphone system (PHS), a personal digital assistant (PDA), international mobile telecommunication-2000 (IMT-2000), code division multiple access-2000 (CDMA-2000), w-code division multiple access (W-CDMA), a wireless broadband internet (WiBro) terminal, and a smart phone, and wearable devices such as a watch, a ring, a bracelet, an anklet, a necklace, glasses, contact lenses, or a head-mounted device (HMD).

'Antigen' in the present specification is a substance that induces an immune response.

A neoantigen is new proteins formed in a cancer cell when a specific mutation occurs in tumor DNA.

The neoantigen is characterized by being caused by the mutation and manifested only in the cancer cell.

The neoantigen may include a polypeptide sequence or a nucleotide sequence. The mutation may include a frameshift or non-lattice shift indel, a missense or nonsense substitution, a splice site alteration, a genomic rearrangement or gene fusion, or any genomic or manifestation alteration causing a new ORF. The mutation may also include a splice variant. A post-translational modification specific to a tumor cell may include an abnormal phosphorylation. The post-translational modification specific to the tumor cell may also include a proteasome-generated spliced antigen.

'Epitope' in the present specification may refer to a specific portion of an antigen to which an antibody or a T-cell receptor is normally bound.

'MHC' in the present specification may be a protein that presents a 'peptide' synthesized in a specific cell on a surface of the cell, thereby enabling a T-cell to identify the cell.

'Peptide' in the present specification is a polymer of amino acids. For convenience of explanation, hereinafter, the 'peptide' is an amino acid polymer or an amino acid sequence represented on a surface of the cancer cell.

'MHC-peptide complex' in the present specification is represented on the surface of the cancer cell and is a complex structure of the MHC and the peptide. The T-cell recognizes the MHC-peptide complex to perform the immune response.

Hereinafter, the operation principles and embodiments of the present disclosure will be described with reference to the accompanying drawings.

Cancer is a disease in which a mutation is manifested in a normal cell to indefinitely proliferate cells, and conventionally, surgery, radiation, and chemotherapy are the main treatments, but, recently, research on an immune anticancer vaccine treatment that utilizes an immune neural network implementation device of a human body has been actively conducted.

The cancer cell creates the neoantigen. The epitope of the neoantigen is represented on major histocompatibility complexes (MHC) located on the surface of the cancer cell. The T-cell recognizes MHC-epitope to cause the immune response.

Therefore, it is necessary to predict a MHC-peptide binding to identify the neoantigen created by the cancer cell.

Specifically, the cancer cell fragments an abnormal peptide created by a gene (a mutation), and abnormal peptide fragments are represented on the surface of the cell by the MHC. When the T-cell recognizes the abnormal peptide fragments, the immune response that destroys the corresponding cell occurs.

Therefore, when the abnormal peptide fragment among abnormal substances generated by the cancer cell, which may move to the surface of the cell and may be bound to the T-cell, is found, directly synthesized, and injected into a patient, cancer treatment is possible because the maturation and proliferation of an immature T-cell that may recognize the neoantigen by an antigen-presenting cell are accelerated.

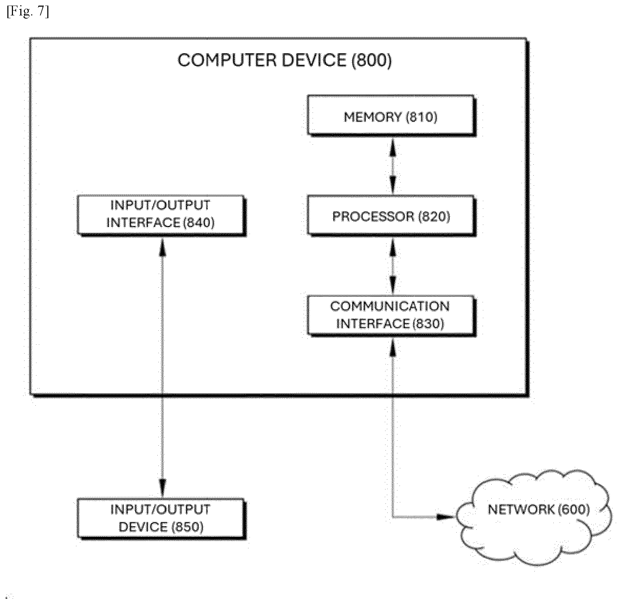

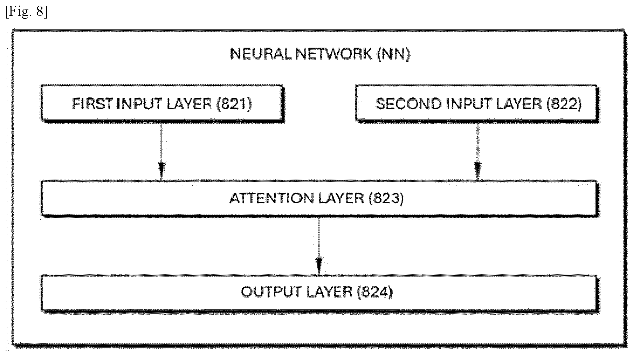

The present disclosure is intended to implement a sequence transduction neural network through training and to predict whether to bind the MHC to a peptide sequence and the activation of the T-cell based on the implemented sequence transduction neural network. A series of operations or algorithms therefor may be performed by a computer device, and a specific configuration of the computer device will be described below based on FIGS. 7 and 8. The computer device in the present specification may be a neural network implementation device that forms a neural network.

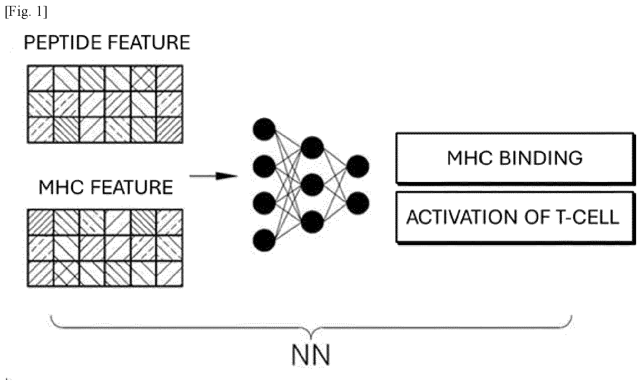

FIG. 1 is a diagram schematically showing a training operation for implementing a sequence transduction neural network NN according to one embodiment of the present disclosure.

Referring to FIG. 1, when training the sequence transduction neural network NN according to one embodiment of the present disclosure, training is performed using pre-stored open data.

Specifically, after an MHC feature 110 is input as first input data and a peptide feature 120 is input as second input data, each is input as input data. Whether MHC binding is possible and whether the T-cell is activated accordingly are trained.

Therefore, the sequence transduction neural network NN for transducing an input sequence may be implemented.

Here, the pre-stored open data used for training may include whether the MHC binding is possible and whether the T-cell is activated depending on the type of the MHC of each peptide as data. Detailed descriptions thereof will be given below.

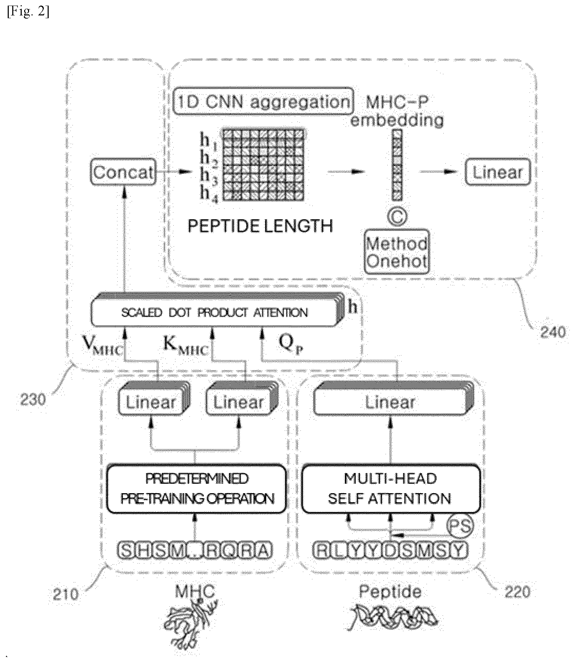

FIG. 2 is a diagram schematically showing an attention algorithm of a sequence transduction neural network implemented according to one embodiment of the present disclosure.

Referring to FIG. 2, when the first input data is input, the computer device performs predetermined pre-training to determine a first key value and a first value value for the first input data (210), and when the second input data corresponding to the first input data is input, the computer device performs multi-head self attention to generate a first query value for the first input data (220).

Next, based on the first key value, the first value value, and the first query value, the computer device performs a scaled dot product attention operation and concatenates each attention head to output a matrix in which each sequence is transduced into a vector (S230). Next, the computer device performs a convolutional neural network (CNN) aggregation operation according to a peptide length, performs an embedding to transduce the matrix into a vector, generates and outputs output data that has passed through a linear layer (240).

In this case, the output data may be data that extracts (filters) only at least one MHC candidate that may activate the T-cell through binding with the T-cell.

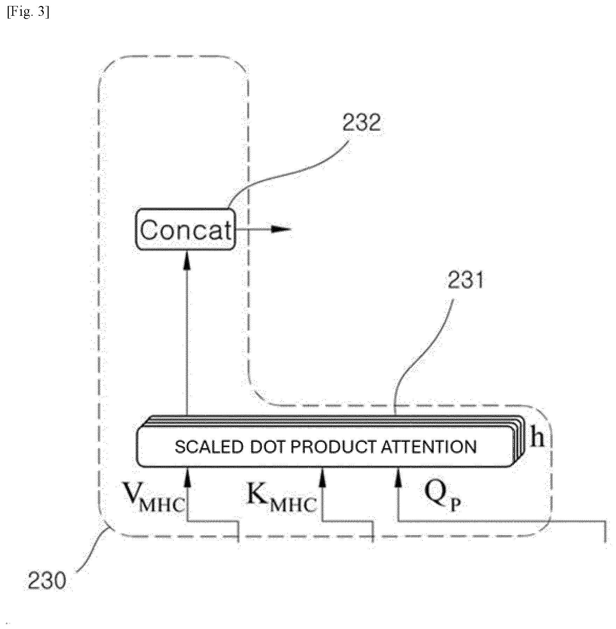

FIG. 3 is a diagram showing an example of performing a scaled dot attention operation in the attention algorithm of the sequence transduction neural network implemented according to one embodiment of the present disclosure, and hereinafter, a processing operation in 230 of FIG. 2 will be described in detail.

Referring to FIG. 3, the computer device may perform self attention based on the first key value, the first value value, and the first query value of the first input data to output a attention score value as an output value (231), and perform concatation of all attention heads (a plurality of attention heads) thereof (232).

Here, the self attention is for extracting the relationship between three elements: a key, a value, and a query and may be the scaled dot product attention operation.

In this case, the computer device calculates the first query value for each sequence of the first input data for all first key values as the attention score value and obtains a probability distribution in which the total value becomes 1 by applying a softmax function.

This is referred to as an attention distribution, and each value is referred to as an attention weight value.

The computer device may calculate an attention value by performing a weighted sum of the attention weight value and a hidden state for each sequence and create a single vector by concatenating the attention value with a hidden state at time t.

That is, the computer device may perform an operation of combining a plurality of attention heads generated through a preset operation, that is, the softmax function based on the first key value, the first value value, and the first query value that correspond to each sequence of the first input data and the second input data.

In this case, the attention weight value corresponds to attention energy for the second input data corresponding to the first input data.

Therefore, an attention value matrix having all the attention values for each sequence of the first input data and the second input data is calculated as the result. This may be represented as shown in Equation 1 below.

[Equation 1]

$$\text{Attention}(Q,K,V) = \text{softmax}\left(\frac{QK^T}{\sqrt{d_k}}\right)V$$

Equation 1 refers to the attention operation, and in Equation 1, Q denotes a query that performs the attention operation, K denotes a key, and V denotes a value corresponding to the query and the key.

The softmax function is an activation function used in multi-classes and may be a function that receives an N-dimensional vector and estimates the probability in which the N-dimensional vector belongs to each class when a class is provided as N classes. The softmax function may be defined as shown in Equation 2 below.

[Equation 2]

$$y_k = \frac{e^{a_k}}{\sum_{i=1}^{n} e^{a_i}}$$

Equation 2 refers to the softmax function, where n denotes the number of neurons in an output layer, and k denotes the order of the classes.

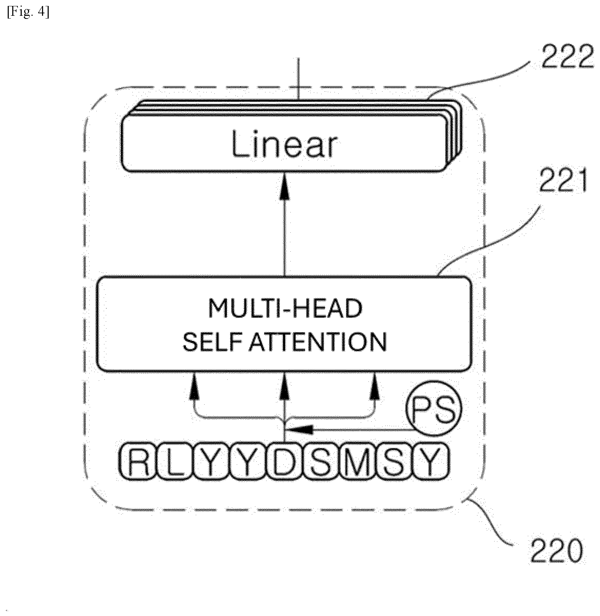

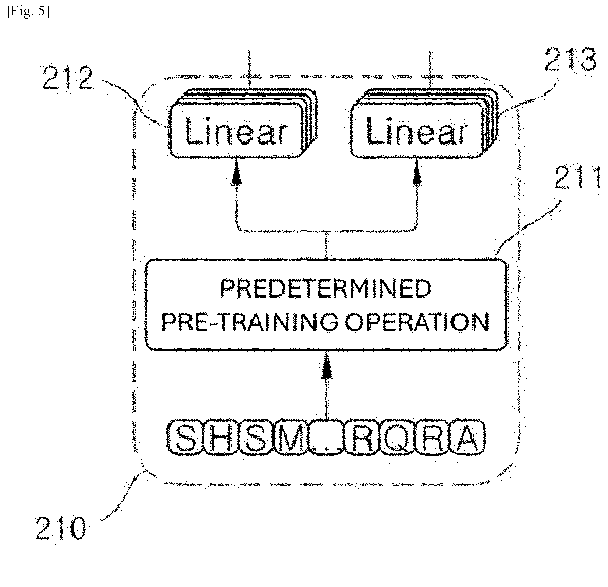

Meanwhile, the first key value and the first value value for the first input data are determined from the first input data, but the first query value may be generated from the second input data, and detailed description thereof will be specifically described below based on FIGS. 4 and 5.

Meanwhile, the output data may be implemented as data that indicates the degree of matching between sequences according to the attention score value calculated by performing the self attention in the attention algorithm of the sequence transduction neural network.

Specifically, the output data may be generated as output data in which the T-cell immunogenicity is matched to each sequence of output data corresponding to the first input data and may be generated by performing a normalization operation on the T-cell immunogenicity corresponding to the sequence of the output data to generate label information corresponding to the second input data.

FIG. 4 is a diagram showing an example of processing second input data in the attention algorithm of the sequence transduction neural network implemented according to one embodiment of the present disclosure, and hereinafter, a processing operation in 220 of FIG. 2 will be described in detail.

Referring to FIG. 4, when the second input data corresponding to the first input data is input into the implemented sequence transduction neural network, the computer device matches position information to each sequence of the second input data. **In** this case, the second input data is a peptide feature (sequence), and both a physicochemical property (AAindex) and an amino acid substitution matrix (BLOSUM) are used as the peptide feature. For example, the second input data may include 9-10 sequences.

Thereafter, a second key value, a second value value, and a second query value that correspond the second input data, that is, for each sequence of the second input data, are generated by the operation with the attention weight through input embedding, and a predetermined self attention operation is performed based on the second key value, the second value value, and the second query value (221). In this case, the attention weight may be operated through pre-training.

Here, the self attention is multi-head attention and may perform num_heads parallel attentions on the second key value, the second value value, and the second query value that have a dimension in which the dimension of the $d_{model}$ is divided into num_heads and may be performed by concatenating all of the attention heads (each attention value matrix) may be performed.

In this case, different attention weights $W_Q$, $W_K$, and $W_V$ are each imparted to attention heads. Thereafter, a value obtained by multiplying a matrix concatenating all of the attention heads by another weight matrix $W_O$ is output as a final result value for the multi-head attention.

Thereafter, the output values pass through a linear layer to generate the first query value for the first input data.

FIG. 5 is a diagram showing an example of processing first input data in the attention algorithm of the sequence transduction neural network implemented according to one embodiment of the present disclosure, and hereinafter, a processing operation in 210 of FIG. 2 will be described in detail.

Referring to FIG. 5, when the first input data is input into the implemented sequence transduction neural network, the computer device performs a predetermined pre-training operation (211) and determines the first key value and the first value value as each output value passes through the linear layer (212 and 213).

In this case, the first input data may include at least one of pieces of information about the type of the MHC and the structure of the MHC.

Specifically, the first input data is an MHC feature (three-dimensional structure), and the MHC feature may use 181 sequences close to a binding site, and as an example, the type of the MHC may be changed to 360 sequences. Meanwhile, the first input data may be provided as a sequence corresponding to a predetermined range based on a point at which the peptide sequence is bound to the MHC sequence.

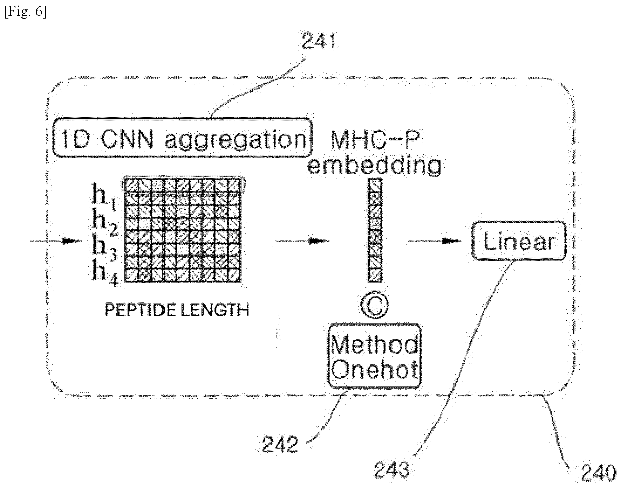

FIG. 6 is a diagram showing an example of generating output data using an attention score value calculated in the attention algorithm of the sequence transduction neural network implemented according to one embodiment of the present disclosure, and hereinafter, a processing operation in 240 of FIG. 2 will be described in detail.

Referring to FIG. 6, when the matrix in which each sequence has been transduced into a vector is output by 230 of FIG. 2, the computer device performs a CNN aggregation operation according to a peptide length (S241) and performs embedding to transduce the matrix into a vector (242). That is, embedding vectors for the first input data and the second input data are generated. Next, output data that has passed through the linear layer is generated and output (243). In this case, information corresponding to an experimental method may additionally be combined with the embedding vector through onehot encoding to generate output data.

FIG. 7 is a diagram showing a configuration of a computer device for transducing an input sequence according to one embodiment of the present disclosure.

Referring to FIG. 7, a computer device 800 may include a memory 810, a processor 820, a communication interface 830, an input/output interface 840, and an input/output device 850. However, each component is shown as one component in FIG. 7, but this is only for convenience of explanation, and at least one component may be provided as needed.

The memory 810 stores data and/or various types of information that support various functions of the computer device 800. The memory 810 may store a plurality of application programs (or applications) that are driven on the computer device 800, and data and commands for operating the computer device 800. At least some of the application programs may be downloaded from an external server via wireless communication. Meanwhile, the application program may be stored in the processor 820, installed on the computer device 800, and driven to perform an operation (or a function) by the processor 820.

Specifically, the memory 810 stores at least one machine learning model and at least one process to implement the sequence transduction neural network for transducing the input sequence.

Meanwhile, the memory 810 may include at least one type of storage medium such as a flash memory type, a hard disk type, a multimedia card micro type, a card-type memory (e.g., an SD or XD memory), a random access memory (RAM), a static RAM (SRAM), a read-only memory (ROM), an electrically erasable programmable ROM (EEPROM), a programmable ROM (PROM), a magnetic memory, a magnetic disk, or an optical disk. In addition, the memory may store information temporarily, permanently, or semi-permanently and may be provided as a built-in or detachable type.

The processor 820 may control all components in the computer device 800 to process signals, data, information, etc. that are input or output, or execute commands, algorithms, and application programs that are stored in the memory 810 to perform various processes, and provide appropriate information or functions to each user or process the same by implementing the sequence transduction neural network for transducing the input sequence.

The processor 820 may perform a predetermined pre-training operation on the basis of first input data to determine a first key value and a first value value, receive the second input data corresponding to the first input data, match position information to each sequence of the second input data, perform a predetermined self attention operation on the basis of a second key value, a second value value, and a second query value that correspond to the second input data to generate a first query value, and perform the predetermined attention operation on the basis of the first key value, the first value value, and the first query value to determine output data corresponding to the first input data and the second input data.

In addition, the processor may perform an operation of combining a plurality of attention heads generated through a predetermined operation on the basis of the first key value, the first value value, and the first query value that correspond to each sequence of the first input data and the second input data.

The operation of combining the attention heads may be defined as a concatenation operation.

The concatenation operation may refer to a function that concatenates a plurality of text strings into a single text string.

The processor may be configured to output the output data including the attention energy for the second input data corresponding to the first input data.

The output data may be generated as output data in which T-cell immunogenicity is matched to each sequence of the output data corresponding to the first input data.

Meanwhile, the neural network may perform a normalization operation on the T-cell immunogenicity corresponding to the sequence of the output data to generate label information corresponding to the second input data.

When the neural network predicts the binding information of the peptide sequence and the MHC, the output data may include the binding information corresponding to the first input data and the second input data.

The binding information may include at least one of binding probability, the presence or absence of binding, binding strength, dissociation constant, and IC50.

On the other hand, when the neural network predicts the activation of the T-cell corresponding to the peptide sequence, the output data may include the probability of the activation of the T-cell corresponding to the first input data and the second input data.

Meanwhile, the first input data according to one embodiment of the present invention may include at least one of pieces of information about the type of the MHC and the structure of the MHC.

In addition, the second input data includes a plurality of peptide sequences.

Meanwhile, when the second input data includes at least one peptide sequence that is not bound to the MHC, the sequence transduction neural network implemented in the neural network implementation device may predict the binding information of the MHC and the peptide sequence.

On the other hand, when the second input data includes at least one peptide sequence that does not activate the T-cell, the sequence transduction neural network may be implemented to predict the probability of the activation of the T-cell.

Specifically, referring to Table 1 below, when the second input data includes a peptide sequence that is negative for MHC binding, the sequence transduction neural network may predict the binding information of the peptide sequence and the MHC.

On the other hand, referring back to Table 1, when the second input data includes the peptide sequence that is negative for the activation of the T-cell, the sequence transduction neural network may predict the probability of the activation of the T-cell.

[Table 1]

| PEPTIDE | MHC TYPE | MHC BINDING (IC50) | ACTIVATION OF T-CELL |
|---------|----------|--------------------|--------------------|
| MGQIVTMFE | A*0201 | = 421 nM | Positive |
| | B*0101 | > 20000 nM | NaN |
| GQIVTMFEA | A*0101 | < 100 nM | Negative |
| MFEALPHII | A*0204 | = 12000 nM | NaN |

The above-described Table 1 is a diagram showing an example of data used for neural network training according to one embodiment of the present invention. The data used for training may include the peptide sequence matched with Kd. Kd denotes the dissociation constant of an antigen binding site. The lower the Kd value may mean the higher the affinity of the antibody for the antigen, and the higher the Kd value may mean the lower the affinity.

Meanwhile, when the binding information of the peptide sequence and the MHC corresponding to the first input data, which are included in the output data corresponding to the first input data, is less than a predetermined value, the processor may modify the variable of the sequence transduction neural network using a loss function determined based on the binding information.

Here, the binding information, which will be predicted, may be determined based on the above-described Kd.

Meanwhile, Kd may be matched in a form of a specific number, but may also be matched in a specific range (inequality).

Each Kd can be used as label information when the neural network operates as a model that predicts the binding information of the MHC and the peptide sequence, and the neural network may reflect the range of the label information in the loss function.

In particular, in the case that training is performed using the peptide sequence with a specific range of Kd, when the Kd of the output data corresponding to the input data is included within the corresponding range, it may be determined that the model has no loss, and the training may be performed.

For example, in the case that training is performed with the peptide sequence 'GQIVTMFEA' and the MHC type 'A*0101,' when the Kd output by the neural network is 97 nM, the corresponding Kd is included in the label information '< 100 nM,' and thus it may be determined that the neural network has no loss, and the training may be performed.

According to another embodiment, in the case that training is performed with the peptide sequence 'MGQIVTMFE' and MHC type 'B*0101,' when the Kd output by the neural network is 20003 nM, the corresponding Kd is included in the label information '> 20000 nM,' and thus it may be determined that the neural network has no loss, and the training may be performed.

In summary, the label information of training data used for training in the present disclosure may include a specific value and a specific range. When the label information is a specific range and a value predicted by the neural network is included in the specific range, it may be determined that the neural network has no loss, and the training may be performed.

Meanwhile, the above-described operation is only one embodiment of the present invention, and there is no limitation on the form of preprocessed data used by the neural network for training.

Meanwhile, the second input data may include at least one of the amino acid substitution matrix (BLOSUM) and the physicochemical property (AAindex) that correspond to the peptide sequence.

In addition, the first input data may be provided as a sequence corresponding to a predetermined range based on the point at which the peptide sequence is bound to the MHC sequence.

In addition, the at least one processor may generate the output data in which the T-cell immunogenicity is matched to each sequence of the output data corresponding to the first input data and perform a normalization operation on the T-cell immunogenicity corresponding to the sequence of the output data to generate label information corresponding to the second input data.

In addition, the processor may generate the output data by additionally combining information corresponding to an experimental result with an embedding vector determined by performing the predetermined attention operation on the basis of the first key value, the first value value, and the first query value.

Meanwhile, the computer device 800 may include one or more components that enable communication with an external device and may, for example, have the communication interface 830 for wireless communication and the input/output interface 830 for wired communication.

Specifically, the communication interface 830 may transmit and receive signals to and from the external device via a network 600 based on wireless communication. To this end, the communication interface 830 may include at least one wireless communication module, short range communication module, and the like.

First, the wireless communication module may include wireless communication modules that support various wireless communication methods such as global system for mobile communication (GSM), code division multiple access (CDMA), wideband code division multiple access (WCDMA), universal mobile telecommunications system (UMTS), time division multiple access (TDMA), wireless local area network (WLAN), digital living network alliance (DLNA), wireless broadband (WiBro), worldwide interoperability for microwave access (WiMAX), high-speed downlink packet access (HSDPA), high-speed uplink packet access (HSUPA), long-term evolution (LTE), 4G, 5G, and 6G in addition to a Wi-Fi module and a wireless broadband module.

In addition, the short range communication module is configured for short range communication and may support short range communication using at least one of Bluetooth™, radio frequency identification (RFID), infrared data association (IrDA), ultra-wideband (UWB), ZigBee, near field communication (NFC), wireless-fidelity (Wi-Fi), Wi-Fi direct, and wireless universal serial bus (Wireless USB) techniques.

Meanwhile, the input/output interface 240 may be connected to the input/output devices 250 in a wired manner, that is, based on wired communication, to transmit and receive signals. To this end, the input/output interface 840 may include at least one wired communication module, and the wired communication module may include not only various wired communication modules such as a local area network (LAN) module, a wide area network (WAN) module, or a value-added network (VAN) module, but also various cable communication modules such as universal serial bus (USB), high definition multimedia interface (HDMI), digital visual interface (DVI), recommended standard 232 (RS-232), powerline communication, or plain old telephone service (POTS).

However, since the components shown in FIG. 7 are not essential for implementing the computer device according to the present disclosure, the implementation device described in the present specification may include a larger number of or fewer number of components than the components listed above. That is, the present disclosure is not limited to the number, names, operations, etc. of the components.

FIG. 8 is a diagram showing a specific configuration of a processor in the computer device of FIG. 7.

Referring to FIG. 8, the processor of the computer device 800 may implement a neural network NN. The neural network NN may include a first input layer 821, a second input layer 822, an attention layer 823, and an output layer 824.

Referring to FIG. 8, the first key value and first value value are determined and output from the first input data input through the first input layer 821, and the second input layer 822 generates the first query value from the second input data corresponding to the first input data.

Thereafter, the attention layer 823 performs self attention based on the first key value, the first value value, and the first query value, and the output layer 824 generates and outputs the output data using the output value.

When the neural network NN operates as a model that predicts the binding of the MHC and the peptide sequence, the output layer 824 may output the output data including the MHC binding information corresponding to the first input data and the second input data.

That is, the output layer 824 may include the binding information of the MHC input as the first input data and the peptide sequence input as the second input data.

The loss function may be determined based on the difference between the binding information included in the output data and the label corresponding to specific first input data and second input data.

Based on this, training that may predict the peptide sequence that may be bound to the MHC may be performed by modifying each variable that constitutes the neural network NN in the neural network implementation device.

Meanwhile, when the neural network NN operates as a model that predicts the activation of the T-cell corresponding to the MHC and the peptide sequence, the output data output from the output layer 824 may include whether to activate the T-cell corresponding to the first input data and the second input data.

Specifically, the output layer 824 may output the output data in the form of a positive value when the T-cell is activated or a negative value when the T-cell is not activated.

According to one embodiment of the present invention, by using a beta distribution of the case that the neural network NN operates as a model that predicts the activation of the T-cell corresponding to the MHC and the peptide sequence, the label information of the corresponding data may be generated.

In order to label the data required for training, the processor may perform a plurality of tests that output whether to activate the T-cell corresponding to the first input data and the second input data.

Meanwhile, the processor according to one embodiment of the present invention may perform the plurality of tests based on the same first input data and second input data.

The neural network NN may output a value of whether to activate the T-cell corresponding to the first input data and the second input data.

The processor in which the neural network is implemented, may determine the number of plurality of tests and the number of times the T cell corresponding to first input data and second input data is activated.

The processor implementing the neural network may generate the beta distribution corresponding to the first input data and the second input data on the basis of the number of times the T cell is activated for the number of plurality of tests.

That is, the processor may output the activation of the T cell for the first input data and the second input data as the beta distribution.

The beta distribution may be a continuous probability distribution defined in section [0, 1] according to two parameters $\alpha$ and $\beta$. In the above definition, the parameter may be a parameter that determines the shape of the distribution.

The probability density function of the beta distribution may be defined as shown in Equation 3 below.

[Equation 3]

$$F(x;a,b) = \frac{1}{B(a,b)} x^{a-1}(1-x)^{b-1}$$

Referring to Equation 3, f denotes the beta distribution, and here, B (a, b) denotes the beta function, which may be defined as shown in Equation 4 below.

[Equation 4]

$$B(a,b) = \int_0^1 t^{a-1}(1-t)^{b-1} dt$$

Meanwhile, the processor driving the neural network may determine the mean and variance of the above beta distribution.

In addition, the processor may determine the label information corresponding to the first input data and the second input data on the basis of the mean and variance of the beta distribution. Specifically, the processor may determine that the difference between the mean and the variance is the label information corresponding to the first input data and the second input data.

Typically, when a large number of tests are performed on such data distribution, a variance value may decrease, and when the number of tests is small, the variance value may increase.

Based on this, the label corresponding to the first input data and the second input data, that is, the MHC and the peptide sequence, may be determined as shown in Equation 5 below.

[Equation 5]

$$L = E - V$$

Referring to Equation 5, L denotes the label information determined by the processor, E denotes the mean of the beta distribution, and V denotes the variance of the beta distribution.

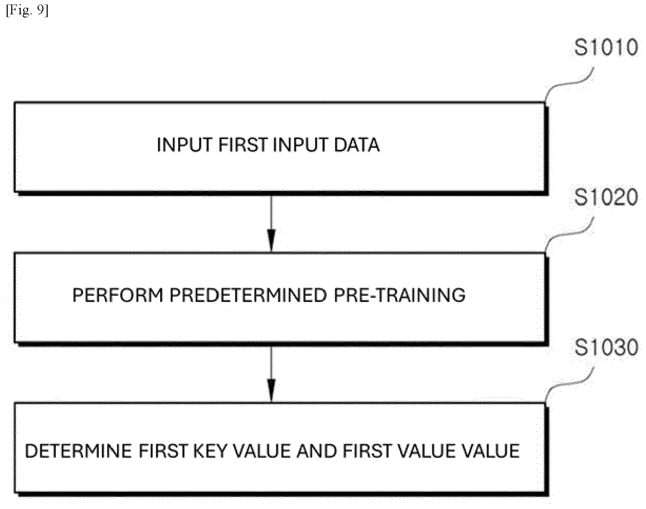

Meanwhile, the operation of the layer constituting each of the neural networks illustrated in FIG. 8 is only one embodiment of the present disclosure, and the present invention is not limited thereto. FIG. 9 is a flowchart showing an example of processing first input data based on the attention algorithm of the sequence transduction neural network implemented according to one embodiment of the present disclosure.

Referring to FIG. 9, when the first input data is input into the implemented sequence transduction neural network (S1010), the computer device 800 performs a predetermined pre-training operation (S1020). Next, as each output value passes through a linear layer, the first key value and the first value value are determined (S1030).

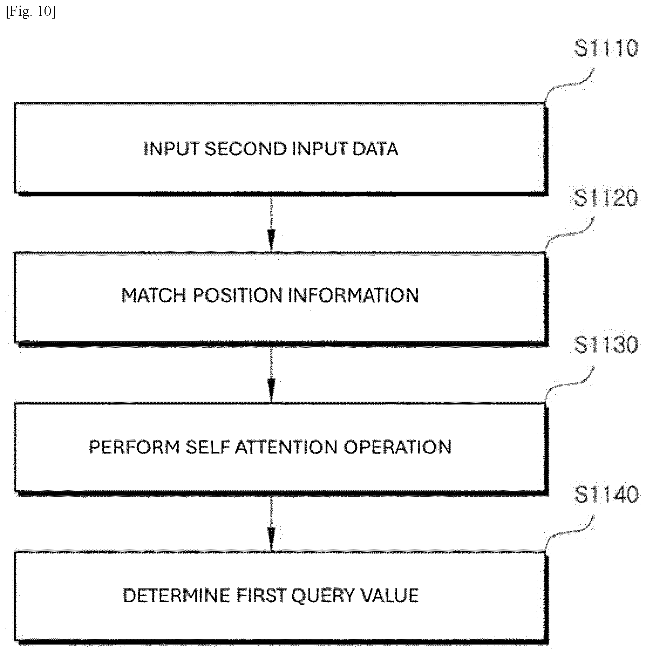

FIG. 10 is a flowchart showing an example of processing second input data based on the attention algorithm of the sequence transduction neural network implemented according to one embodiment of the present disclosure.

Referring to FIG. 10, when the second input data corresponding to the first input data is input into the implemented sequence transduction neural network (S1110), the computer device 800 matches position information to each sequence of the second input data (S1120).

Next, the second key value, the second value value, and the second query value that correspond to the second input data, that is, each sequence of the second input data, are generated by operating the attention weight through input embedding, and a predetermined self attention operation is performed based on the second key value, the second value value, and the second query value (S1130).

Next, the output values pass through the linear layer to generate the first query value for the first input data (S1140).

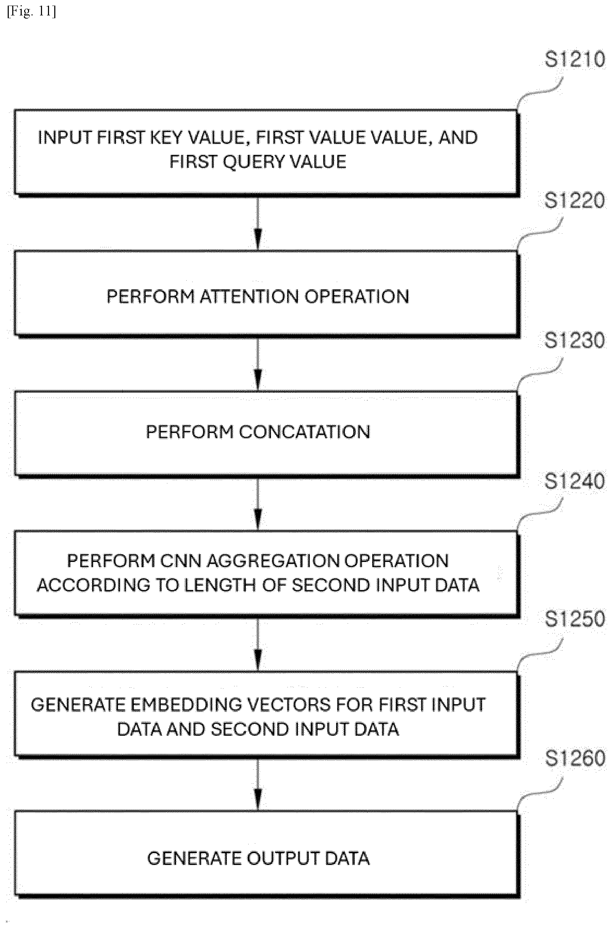

FIG. 11 is a flowchart showing an example of generating output data using an attention score value calculated based on the attention algorithm of the sequence transduction neural network implemented according to one embodiment of the present disclosure.

Referring to FIG. 11, when the first key value, the first value value, and the first query value of the first input data are input (S1210), the self attention may be performed on the basis of the first key value, the first value value, and the first query value to output the attention score value as an output value (S1220) and concatation of all attention heads may be performed (S1230).

Next, the CNN aggregation operation is performed according to a length of the second input data, that is, a length of the peptide sequence (S1240), and embedding vectors for the first input data and the second input data are generated (S1250).

Next, the output data that has passed through the linear layer is generated, and the output data is output (S1260).

As described above, the output data may include the binding information of the MHC and the peptide sequence or the probability of the activation of the T-cell.

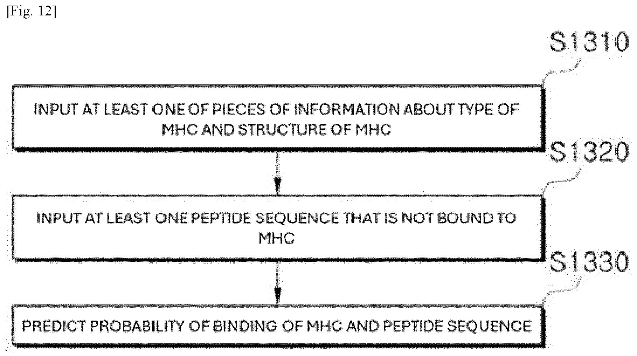

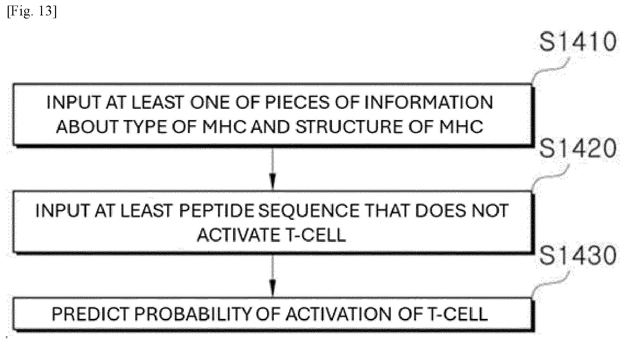

FIGS. 12 and 13 are flowcharts showing operations that predict the binding information of the MHC and the peptide sequence and whether to activate the T-cell corresponding to the peptide sequence based on the attention algorithm of the sequence transduction neural network implemented according to one embodiment of the present disclosure.

FIG. 12 shows an operation of a neural network that predicts the binding information of the MHC and the peptide sequence.

Referring to FIG. 12, the first input data and the second input data are input into the neural network. In FIG. 12, pieces of information about the type of the MHC and the structure of the MHC may be input as the first input data (S 1310).

In addition, the second input data may include the peptide sequence. When the neural network predicts the binding information of the MHC and the peptide sequence as in FIG. 12, the second input data may include at least one peptide sequence that is not bound to the MHC (S1320).

In the case of FIG. 12, the neural network may receive the pieces of information about the type of the MHC and the structure of the MHC as the first input data and receive a plurality of peptide sequences as the second input data (S1320).

Thereafter, the neural network that has received the first input data and the second input data may perform the attention operation using the query, the key, and the value that are derived from each data and predict the binding information of the MHC and the peptide sequence (S1330).

FIG. 13 shows an operation of a neural network that predicts whether to activate the T-cell corresponding to the peptide sequence.

Referring to FIG. 13, the first input data and the second input data are input into the neural network.

In the case of FIG. 13, the neural network may receive pieces of information about the type of the MHC and the structure of the MHC as the first input data and receive a plurality of peptide sequences as the second input data (S 1410 and S 1420).

Here, the second input data may include both of the peptide sequence that activates the T-cell and the peptide sequence that does not activate the T-cell (S 1420).

The neural network that has received the first input data and the second input data may perform the attention operation using the query, the key, and the value that are derived from each data and predict the probability of the activation of the T-cell (S1430).

Meanwhile, disclosed embodiments may be implemented in the form of a recording medium in which computer-executable commands are stored. The commands may be stored in the form of program code, and when executed by a processor, program modules are generated to perform operations of the disclosed embodiments. The recording medium may be implemented as a computer-readable recording medium.

The computer-readable recording medium includes all types of recording media in which computer-decodable commands are stored. For example, there may be a ROM, a RAM, a magnetic tape, a magnetic disk, a flash memory, an optical data storage device, and the like.

As described above, the disclosed embodiments have been described with reference to the accompanying drawings. Those skilled in the art to which the present disclosure pertains will understand that the present disclosure may be implemented in different forms from the disclosed embodiments without departing from the technical spirit or essential features of the present disclosure. The disclosed embodiments are illustrative and should not be construed as limiting.


**Claims**

1. A neural network implementation device for implementing a sequence transduction neural network for transducing an input sequence having each network input for at least one computer, comprising:

    at least one memory; and
    at least one processor configured to communicate with the at least one memory,
    wherein the at least one processor is configured to:

        receive first input data;
        receive second input data corresponding to the first input data;
        train a sequence transduction neural network by performing an attention operation on the basis of the first input data and second input data labeled with predetermined label information; and
        determine output data that is output by the sequence transduction neural network trained on the basis of the first input data, the second input data, and the label information.

2. The neural network implementation device of claim 1, wherein the at least one processor is configured to:

        perform a predetermined pre-training operation on the basis of the first input data to determine a first key value and a first value value;
        match position information to each sequence of the second input data;
        performs a predetermined self attention operation on the basis of a second key value, a second value value, and a second query value corresponding to the second input data to generate a first query value; and
        perform the predetermined attention operation on the basis of the first key value, the first value value, and the first query value to determine the output data corresponding to the first input data and the second input data.

3. The neural network implementation device of claim 2, wherein the at least one processor performs an operation of combining a plurality of attention heads generated through a predetermined operation on the basis of the first key value, the first value value, and the first query value that correspond to each sequence of the first input data and the second input data.

4. The neural network implementation device of claim 2, wherein the at least one processor outputs the output data including attention energy for the second input data corresponding to the first input data.

5. The neural network implementation device of claim 4, wherein the at least one processor generates the output data by additionally combining information corresponding to an experimental method with an embedding vector determined by performing the predetermined attention operation on the basis of the first key value, the first value value, and the first query value.

6. The neural network implementation device of claim 2, wherein the first input data includes at least one of pieces of information about a type of MHC and a structure of the MHC, and
the second input data includes a plurality of peptide sequences.

7. The neural network implementation device of claim 6, wherein the sequence transduction neural network is implemented to predict whether the MHC is bound to the peptide sequence when the second input data includes at least one peptide sequence that is not bound to the MHC.

8. The neural network implementation device of claim 6, wherein the at least one processor modifies a variable of the sequence transduction neural network using a loss function determined on the basis of binding information of the peptide sequence and the MHC that correspond to the first input data and the second input data that are included in the output data.

9. The neural network implementation device of claim 8, wherein the second input data includes at least one of an amino acid substitution matrix (BLOSUM) and a physicochemical property (AAindex) corresponding to the peptide sequence.

10. The neural network implementation device of claim 7, wherein the first input data is provided as a sequence corresponding to a predetermined range based on a point at which the peptide sequence is bound to the MHC.

11. The neural network implementation device of claim 6, wherein the sequence transduction neural network is implemented to predict whether to activate a T-cell matched to the peptide sequence when the second input data includes at least one peptide sequence that does not activate the T-cell.

12. The neural network implementation device of claim 11, wherein the at least one processor is configured to:

    perform a plurality of tests that output whether to activate the T-cell corresponding to the first input data and the second input data;
    determine the number of plurality of tests and the number of times the T-cell is activated;
    generate a beta distribution corresponding to the first input data and the second input data on the basis of the number of times the T-cell is activated for the number of plurality of tests; and
    determine label information corresponding to the first input data and the second input data on the basis of a mean and variance of the beta distribution.

13. A training method for transducing an input sequence performed by a sequence transduction neural network on a computer device, comprising:

    performing a predetermined pre-training operation on the basis of first input data to determine a first key value and a first value value;
    matching position information to each sequence of second input data when the second input data corresponding to the first input data is input;
    performing a predetermined self attention operation on the basis of a second key value, a second value value, and a second query value that correspond to the second input data to generate a first query value; and
    performing the predetermined attention operation on the basis of the first key value, the first value value, and the first query value to determine the output data corresponding to the first input data and the second input data.

14. A computer-readable recording medium which is coupled to a computer that is hardware and in which a program for performing the artificial intelligence-based sequence transduction neural network of claim 13 is stored.

[Fig. 1]

[Fig. 2]

[Fig. 3]

[Fig. 4]

[Fig. 5]

[Fig. 6]

241

1D CNN aggregation   MHC–P embedding

$h_1$
$h_2$
$h_3$
$h_4$

PEPTIDE LENGTH

Method Onehot

Linear

242

243

240

[Fig. 7]

[Fig. 8]

NEURAL NETWORK (NN)

FIRST INPUT LAYER (821)

SECOND INPUT LAYER (822)

ATTENTION LAYER (823)

OUTPUT LAYER (824)

[Fig. 9]

```
                                                    S1010
┌─────────────────────────────────────────┐
│                                           │
│         INPUT FIRST INPUT DATA            │
│                                           │
└─────────────────────────────────────────┘
                     │
                     ▼                       S1020
┌─────────────────────────────────────────┐
│                                           │
│     PERFORM PREDETERMINED PRE-TRAINING    │
│                                           │
└─────────────────────────────────────────┘
                     │
                     ▼                       S1030
┌─────────────────────────────────────────┐
│                                           │
│  DETERMINE FIRST KEY VALUE AND FIRST      │
│             VALUE VALUE                    │
└─────────────────────────────────────────┘
```

[Fig. 10]

INPUT SECOND INPUT DATA — S1110

MATCH POSITION INFORMATION — S1120

PERFORM SELF ATTENTION OPERATION — S1130

DETERMINE FIRST QUERY VALUE — S1140

[Fig. 11]

```
                                              ⌇ S1210
┌─────────────────────────────────────────────┐
│   INPUT FIRST KEY VALUE, FIRST VALUE VALUE, AND│
│            FIRST QUERY VALUE                  │
└─────────────────────────────────────────────┘
                      │
                      ▼                       ⌇ S1220
┌─────────────────────────────────────────────┐
│         PERFORM ATTENTION OPERATION           │
└─────────────────────────────────────────────┘
                      │
                      ▼                       ⌇ S1230
┌─────────────────────────────────────────────┐
│           PERFORM CONCATATION                 │
└─────────────────────────────────────────────┘
                      │
                      ▼                       ⌇ S1240
┌─────────────────────────────────────────────┐
│   PERFORM CNN AGGREGATION OPERATION           │
│   ACCORDING TO LENGTH OF SECOND INPUT DATA    │
└─────────────────────────────────────────────┘
                      │
                      ▼                       ⌇ S1250
┌─────────────────────────────────────────────┐
│  GENERATE EMBEDDING VECTORS FOR FIRST INPUT   │
│        DATA AND SECOND INPUT DATA             │
└─────────────────────────────────────────────┘
                      │
                      ▼                       ⌇ S1260
┌─────────────────────────────────────────────┐
│           GENERATE OUTPUT DATA                │
└─────────────────────────────────────────────┘
```

[Fig. 12]

S1310

INPUT AT LEAST ONE OF PIECES OF INFORMATION ABOUT TYPE OF
MHC AND STRUCTURE OF MHC

S1320

INPUT AT LEAST ONE PEPTIDE SEQUENCE THAT IS NOT BOUND TO
MHC

S1330

PREDICT PROBABILITY OF BINDING OF MHC AND PEPTIDE SEQUENCE

[Fig. 13]

S1410

| INPUT AT LEAST ONE OF PIECES OF INFORMATION ABOUT TYPE OF MHC AND STRUCTURE OF MHC |
|---|

S1420

| INPUT AT LEAST PEPTIDE SEQUENCE THAT DOES NOT ACTIVATE T-CELL |
|---|

S1430

| PREDICT PROBABILITY OF ACTIVATION OF T-CELL |
|---|

## INTERNATIONAL SEARCH REPORT

| International application No. |
| --- |
| **PCT/KR2023/012469** |

| A. | CLASSIFICATION OF SUBJECT MATTER |
| --- | --- |

**G06N 3/08**(2006.01)i; **G06N 3/042**(2023.01)i; **G06N 3/063**(2006.01)i

According to International Patent Classification (IPC) or to both national classification and IPC

| B. | FIELDS SEARCHED |
| --- | --- |

Minimum documentation searched (classification system followed by classification symbols)

G06N 3/08(2006.01); G06F 40/30(2020.01); G06F 40/58(2020.01); G06N 3/04(2006.01); G10L 13/047(2013.01); G10L 13/07(2013.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 멀티-헤드 어텐션 네트워크(multi-head attention network), 셀프 어텐션(self attention), 시퀀스(sequence), 주조직 적합 복합체(major histocompatibility complex, MHC), 펩타이드(peptide)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2021-0174170 A1 (TENCENT TECHNOLOGY (SHENZHEN) COMPANY LIMITED) 10 June 2021 (2021-06-10)<br>See paragraphs [0018], [0078] and [0113]; claims 1 and 8-9; and figures 6-7. | 1-5,13-14 |
| Y | | 6-11 |
| A | | 12 |
| Y | CHENG, Jun et al. BERTMHC: improved MHC–peptide class II interaction prediction with transformer and multiple instance learning. Bioinformatics. Vol. 37, No. 22, pp. 4172–4179, November 2021.<br>See page 4174, figure 1. | 6-11 |
| A | ABDIN, Osama et al. PepNN: a deep attention model for the identification of peptide binding sites. Communications Biology. Vol. 5, No. 503, 26 May 2022.<br>See pages 1-8. | 1-14 |
| A | US 2020-0258496 A1 (TENCENT AMERICA LLC) 13 August 2020 (2020-08-13)<br>See paragraphs [0041]-[0061]; and figures 4-5. | 1-14 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **27 November 2023** | **27 November 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/012469**

### C. DOCUMENTS CONSIDERED TO BE RELEVANT

| Category* | Citation of document, with indication, where appropriate, of the relevant passages | Relevant to claim No. |
|---|---|---|
| A | US 2020-0380213 A1 (SALESFORCE.COM, INC.) 03 December 2020 (2020-12-03) <br> See paragraphs [0036]-[0040]; and figure 5. | 1-14 |

Form PCT/ISA/210 (second sheet) (July 2022)

<table>
<tr><td colspan="2">**INTERNATIONAL SEARCH REPORT**<br>**Information on patent family members**</td><td>International application No.<br>**PCT/KR2023/012469**</td></tr>
</table>

| Patent document<br>cited in search report | Publication date<br>(day/month/year) | Patent family member(s) | Publication date<br>(day/month/year) |
|---|---|---|---|
| US 2021-0174170 A1 | 10 June 2021 | CN 109543824 A | 29 March 2019 |
| | | CN 109543824 B | 23 May 2023 |
| | | WO 2020-108332 A1 | 04 June 2020 |
| US 2020-0258496 A1 | 13 August 2020 | US 11011154 B2 | 18 May 2021 |
| | | WO 2020-163422 A1 | 13 August 2020 |
| US 2020-0380213 A1 | 03 December 2020 | CN 111699498 A | 22 September 2020 |
| | | CN 111699498 B | 13 August 2021 |
| | | CN 111712836 A | 25 September 2020 |
| | | EP 3750111 A1 | 16 December 2020 |
| | | EP 3750112 A1 | 16 December 2020 |
| | | JP 2021-507429 A | 22 February 2021 |
| | | JP 2021-513165 A | 20 May 2021 |
| | | JP 2022-023064 A | 07 February 2022 |
| | | JP 6952201 B2 | 20 October 2021 |
| | | JP 7109557 B2 | 29 July 2022 |
| | | JP 7285895 B2 | 02 June 2023 |
| | | US 10776581 B2 | 15 September 2020 |
| | | US 11501076 B2 | 15 November 2022 |
| | | US 11615249 B2 | 28 March 2023 |
| | | US 2019-0251168 A1 | 15 August 2019 |
| | | US 2019-0251431 A1 | 15 August 2019 |
| | | WO 2019-156873 A1 | 15 August 2019 |
| | | WO 2019-156875 A1 | 15 August 2019 |

Form PCT/ISA/210 (patent family annex) (July 2022)